# EUROPEAN PATENT APPLICATION

(11) **EP 4 467 193 A1**
(43) Date of publication of application: **27.11.2024**
(21) Application number: 23175606.5
(22) Date of filing: 26.05.2023
(51) Int. Cl.: A61P 9/00, A61P 17/02, A61P 29/00, A61P 35/00, C07C 69/76, C07C 219/22, C07D 231/06, C07D 231/12, A61K 31/415

(54) **ENANTIOSELECTIVE PREPARATION METHOD**

(71) Applicant: AnaMar AB, 111 36 Stockholm (SE)
(72) Inventor: PETTERSSON, Lars, 224 80 Lund (SE)
(74) Representative: EIP

(57) **Abstract**

The present application relates to a method for preparing a compound of Formula 1 or Formula 2 or a pharmaceutically acceptable salt thereof. The invention further relates to the compound of Formula 1 or Formula 2 or a pharmaceutically acceptable salt thereof, to a pharmaceutical composition comprising said compound and to the use of said compound as a medicament. Synthetic intermediates are also disclosed.

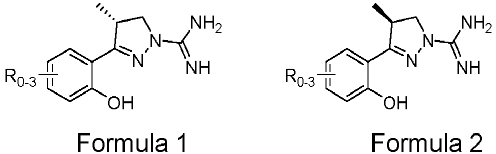

## Description

### Field of the invention

The present invention relates to enantioselective preparation methods for preparing chiral 3-phenyl-4-methyl-4,5-dihydro-1H-pyrazole-1-carboximidamide derivatives, such as (4R)- and (4S)-3-(4-fluoro-2-hydroxyphenyl)-4-methyl-4,5-dihydro-1H-pyrazole-1-carboximidamide, and pharmaceutically acceptable salts thereof.

### Background of the invention

Preparation of 5-HT_{2B} receptor antagonist as anti-fibrotic agents was described in WO2016/207231A1 and compounds were selected for pharmaceutical development. One such selected compound was 3-(3-fluoro-2-hydroxyphenyl)-4-methyl-4,5-dihydro-1H-pyrazole-1-carboximidamide, exemplified by its hydrobromide salt (Example 50 in WO2016/207231A1).

The preparative synthetic methods described in WO2016/207231A1 could only provide racemic mixtures of chiral compounds, and chiral preparative LC methods were needed to prepare samples of the pure enantiomers. To investigate pure chiral compounds for selection and further pharmaceutical development, there is a need for effective preparative enantioselective synthetic methods to avoid ineffective and expensive chiral separation by LC methods.

### Summary of the invention

The two new enantiomeric compounds, (4R)- and (4S)-3-(4-fluoro-2-hydroxyphenyl)-4-methyl-4,5-dihydro-1H-pyrazole-1-carboximidamide, were separated by chiral supercritical fluid chromatography (SFC) and structures of the HCl salts, respectively, were determined by X-ray crystallography (XRD data collected at 100 K with Pilatus 6M-F detector and the structures were solved at 0.75 Å resolution using SHELXS2, SHELXL2, and SHELXLE3 programs).

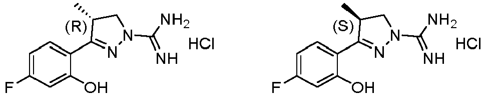

To proceed into clinical studies, there exists need for chemical preparative methods of the new enantiomeric 5-HT_{2B} receptor antagonists (4R)- and (4S)-3-(4-fluoro-2-hydroxyphenyl)-4-methyl-4,5-dihydro-1H-pyrazole-1-carboximidamide hydrochloride, to provide the compounds in larger amounts.

An object of the present application is to find methods for the chemical preparation of chiral 3-phenyl-4-methyl-4,5-dihydro-1H-pyrazole-1-carboximidamide derivatives, and specifically (4R)- and (4S)-3-(4-fluoro-2-hydroxyphenyl)-4-methyl-4,5-dihydro-1H-pyrazole-1-carboximidamide, and pharmaceutically acceptable salts thereof, suitable for large scale production.

The above objects are achieved by the subject matter described herein.

In the present disclosure, a method for preparing chiral 3-phenyl-4-methyl-4,5-dihydro-1H-pyrazole-1-carboximidamide derivatives, such as (4R)- and (4S)-3-(4-fluoro-2-hydroxyphenyl)-4-methyl-4,5-dihydro-1H-pyrazole-1-carboximidamide, based on stereoselective hydrogenation of a key exo-methylene derivative, is described.

Various aspects of the invention are set out in the attached independent claims.

Further advantageous embodiments of the invention are set out in the attached dependent claims.

Applications and advantages of aspects and embodiments of the invention will be apparent from the following detailed description.

### Detailed description of the invention

According to a first aspect of the invention, the above mentioned and other objects are achieved by a method for preparing a compound of Formula 1 or Formula 2 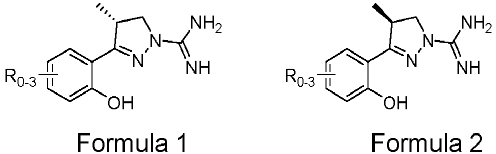
or a pharmaceutically acceptable salt thereof;
wherein R₀₋₃ represents 0-3 aromatic substituents independently selected from methyl, methoxy, F, and Cl;
the method comprising:
   a) providing a compound of Formula 3 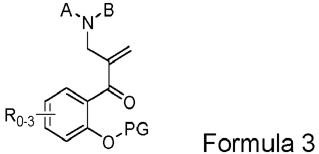 wherein
      A and B are selected from methyl, ethyl, benzyl, or together form a pyrrolidine or piperidine N-heterocycle;
      R₀₋₃ represents 0-3 aromatic substituents independently selected from methyl, methoxy, F, and Cl;
      PG is selected from methyl, benzyl, 4-methoxybenzyl, and tert-butyl-dimethylsilyl;
   b) subjecting the compound of Formula 3 to a ring closure process with aminoguanidine or aminoguanidine hydrochloride to obtain a compound of Formula 4, optionally as a salt with an acid selected from HCl, AcOH, H₂SO₄, H₃PO₄, CF₃CO₂H and MeS(O)₂OH, 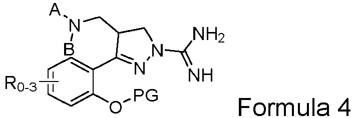 wherein A, B, R₀₋₃, and PG are as described above;
   c) subjecting the compound of Formula 4 to an elimination process to obtain a compound of Formula 5, optionally as a salt with an acid selected from HCl, AcOH, H₂SO₄, H₃PO₄, CF₃CO₂H and MeS(O)₂OH 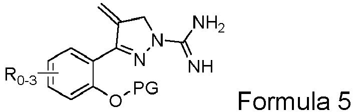 wherein R₀₋₃, and PG are as described above;
   d) subjecting the compound of Formula 5 to enantioselective hydrogenation to obtain a compound of Formula 6 or Formula 7, optionally as a salt with an acid selected from HCl, AcOH, H₂SO₄, H₃PO₄, CF₃CO₂H and MeS(O)₂OH; 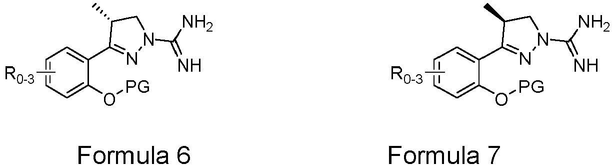 wherein R₀₋₃, and PG are as described above; and
   e) subjecting the compound of Formula 6 or Formula 7 to deprotection, and optionally forming a pharmaceutically acceptable salt of the deprotected compound to obtain the compound of Formula 1 or Formula 2 or a pharmaceutically acceptable salt thereof.

As used herein, the term "pharmaceutically acceptable salt" refers to a chemical compound formed by the reaction between a pharmaceutically active compound and an acid or base, which is suitable for use in pharmaceutical formulations and is generally recognized as safe for human or veterinary administration. The term "pharmaceutically acceptable" indicates that the salt is compatible with the intended pharmaceutical application, including but not limited to solubility, stability, bioavailability, and lack of toxicity or adverse effects. Furthermore, the salt should meet the regulatory requirements set forth by relevant authorities, such as the U.S. Food and Drug Administration (FDA) or the European Medicines Agency (EMA), ensuring its suitability for use in the development, manufacturing, and commercialization of pharmaceutical products. Such salts include acid addition salts, which can be obtained by reaction of the free base of the pharmaceutically active compound with inorganic acids such as hydrochloric acid, hydrobromic acid, nitric acid, phosphoric acid, sulfuric acid, and perchloric acid and the like, or with organic acids such as acetic acid, oxalic acid, (D) or (L) malic acid, maleic acid, methanesulfonic acid, ethanesulfonic acid, p-toluenesulfonic acid, salicylic acid, tartaric acid, citric acid, succinic acid or malonic acid and the like.

In a preferred embodiment the pharmaceutically acceptable salt of the compound of Formula 1 or Formula 2 is the HCl salt of the compound of Formula 1 or Formula 2.

As used herein, the term "aromatic substituents" refers to substituent groups attached to an aromatic ring structure.

As used herein, the term "enantioselective hydrogenation" refers to a chemical process for selectively reducing a prochiral or chiral compound using hydrogen gas (H₂), or alternative hydrogen donor reagents, in the presence of a catalyst to produce a chiral product with a specific desired stereoisomeric configuration. The term "enantioselective" signifies the ability of the hydrogenation process to favor the formation of one enantiomer over the other, leading to the production of a single enantiomer or an enriched mixture of a specific enantiomer. The enantioselective hydrogenation process typically involves the use of a chiral catalyst, which imparts chirality to the reaction, thereby influencing the stereochemical outcome. The catalyst can be a transition metal complex, such as those based on rhodium, ruthenium, or iridium, among others, or other types of catalysts, such as enzymes or organocatalysts. The chiral catalyst selectively interacts with the substrate, facilitating the hydrogenation reaction in a manner that favors the formation of a specific enantiomer, while suppressing or inhibiting the formation of its enantiomeric counterpart.

As used herein, the term "PG" refers to a protecting group. The term "protecting group" refers to a temporary modification or derivatization strategically installed on a reactive site of a molecule to prevent undesired reactions or transformations during synthetic processes, while allowing selective reactions at other sites.

As used herein, the term "deprotection" refers to a chemical process that involves the removal or cleavage of a specific protective moiety or protecting group attached to a functional group of a compound to restore its original reactivity and functionality.

The starting compound, the compound of Formula 3, provided in step a) of the inventive method can be prepared by known methods described in the literature. It is understood that protecting groups (PG) may be employed and that addition, deletion, or transformation of substituents may be part of the synthetic routes.

The enantiomeric purity of the compound of Formula 1 or Formula 2 or a pharmaceutically acceptable salt thereof can, if desired, be further improved by recrystallization of the compounds, preferably as salt derivatives, e.g. HCl salts, or as diastereomeric salts.

In some embodiments, the inventive method further comprises:
f) subjecting the compound of Formula 1 or Formula 2 or a pharmaceutically acceptable salt thereof obtained in e) to diastereomeric salt formation with di-O,O'-p-toloyl-D-tartaric acid or di-O,O'-p-toloyl-L-tartaric acid, respectively, followed by salt exchange to obtain the compound of Formula 1 or Formula 2 or a pharmaceutically acceptable salt thereof.

As used herein the term "diastereomeric salt formation" refers to the formation of salts from a chiral compound and an acid or base, resulting in the generation of diastereomers. Diastereomers are stereoisomers that are not mirror images of one another, but possess different configurations at one or more chiral centers, while maintaining the same connectivity of atoms. The term "diastereomeric salt" indicates that the salt is composed of diastereomers rather than enantiomers.

As used herein the term "salt exchange" refers to a chemical process involving the transformation of a salt from one counterion to another. It involves the exchange of ions within a salt while retaining the overall molecular structure of the compound.

In some embodiments of the inventive method, the obtained compound of Formula 1 or Formula 2 or a pharmaceutically acceptable salt thereof has an enantiomeric purity of at least 80%, preferably at least 90%, and more preferably at least 95%.

As used herein, the term "enantiomeric purity" refers to the prevalence of one enantiomer of a compound over the opposite enantiomer of the compound. A typical optically pure compound comprises greater than about 80% by weight of one enantiomer of the compound and less than about 20% by weight of the opposite enantiomer of the compound, more preferably greater than about 90% by weight of one enantiomer of the compound and less than about 10% by weight of the opposite enantiomer of the compound, even more preferably greater than about 95% by weight of one enantiomer of the compound and less than about 5% by weight of the opposite enantiomer of the compound, and most preferably greater than about 97% by weight of one enantiomer of the compound and less than about 3% by weight of the opposite enantiomer of the compound.

In some embodiments of the first aspect, the compound of Formula 1 or Formula 2 is the compound of Formula 1. In some embodiments, the compound of Formula 1 or Formula 2 is the compound of Formula 2.

The inventive method of the first aspect provides products useful as medicaments, e.g. for the treatment of fibrosis, cardiovascular diseases, pain, IBD, inflammatory diseases, or cancer.

According to a second aspect of the invention, there is provided a compound of Formula 1 or Formula 2 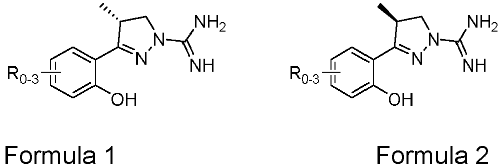 or a pharmaceutically acceptable salt thereof;
wherein R₀₋₃ represents 0-3 aromatic substituents are independently selected from methyl, methoxy, F, and Cl.

In some embodiments of the second aspect, the compound of Formula 1 or Formula 2 or a pharmaceutically acceptable salt thereof has an enantiomeric purity of at least 80%, preferably at least 90%, and more preferably at least 95%. In some embodiments of the second aspect, the compound of Formula 1 or Formula 2 is the compound of Formula 1. In some embodiments, the compound of Formula 1 or Formula 2 is the compound of Formula 2.

In a preferred embodiment the pharmaceutically acceptable salt of the compound of Formula 1 or Formula 2 is the HCl salt of the compound of Formula 1 or Formula 2.

In some embodiments of the second aspect, R₀₋₃ represents 0-2 aromatic substituents independently selected from F, and Cl.

In some embodiments of the second aspect, the compound is selected from: 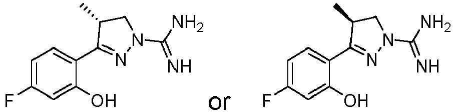 or a pharmaceutically acceptable salt thereof.

In some embodiments of the second aspect, the compound is:
(R)-3-(4-fluoro-2-hydroxyphenyl)-4-methyl-4,5-dihydro-1H-pyrazole-1-carboximidamide hydrochloride; or
(S)-3-(4-fluoro-2-hydroxyphenyl)-4-methyl-4,5-dihydro-1H-pyrazole-1-carboximidamide hydrochloride.

The inventive method of the first aspect described herein further provides a number of intermediates useful for obtaining the desired compounds of Formula 1 or Formula 2 or a pharmaceutically acceptable salt thereof. Each of these intermediates are relevant as starting materials or intermediates for preparing the desired compounds of Formula 1 or Formula 2 or a pharmaceutically acceptable salt thereof. According to a third aspect of the invention, there is provided a compound selected from Formula 3, Formula 4, Formula 5, Formula 6, or Formula 7 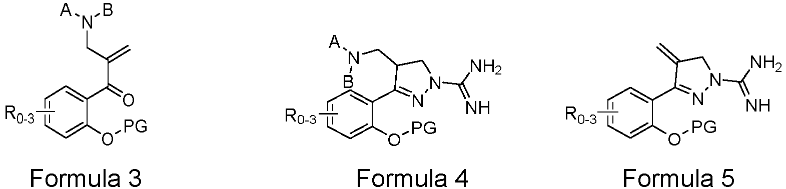 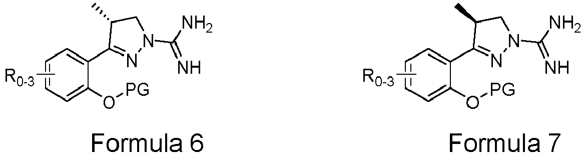
and salts thereof formed with acids selected from HCl, AcOH, H₂SO₄, H₃PO₄, CF₃CO₂H, and MeS(O)₂OH; wherein
A and B are selected from methyl, ethyl, benzyl, or together form a pyrrolidine or piperidine N-heterocycle;
R₀₋₃ represents 0-3 aromatic substituents independently selected from methyl, methoxy, F, and Cl; and
PG is selected from methyl, benzyl, 4-methoxybenzyl, and tert-butyl-dimethylsilyl.

In some embodiments of the third aspect, the compound is selected from Formula 3 or Formula 4, and salts thereof formed with acids selected from HCl, AcOH, H₂SO₄, H₃PO₄, CF₃CO₂H, and MeS(O)₂OH; wherein
A and B are selected from methyl, and benzyl;
R₀₋₃ represents 0-3 aromatic substituents independently selected from F, and Cl; and
PG is benzyl.

In some embodiments of the third aspect, the compound is selected from: 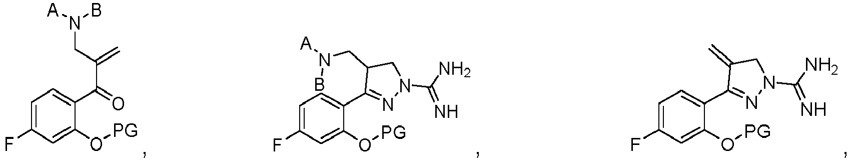 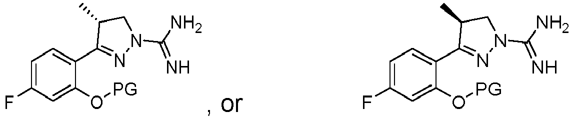
and salts thereof formed with acids selected from HCl, AcOH, H₂SO₄, H₃PO₄, CF₃CO₂H, and MeS(O)₂OH; wherein
A and B are selected from methyl, ethyl, benzyl, or together form a pyrrolidine or piperidine N-heterocycle; and
PG is selected from methyl, benzyl, 4-methoxybenzyl, and tert-butyl-dimethylsilyl.

In some embodiments of the third aspect, the compound is selected from:
1-(2-(Benzyloxy)-4-fluorophenyl)-2-((dimethylamino)methyl)prop-2-en-1-one;
3-(2-(Benzyloxy)-4-fluorophenyl)-4-((dimethylamino)methyl)-4,5-dihydro-1H-pyrazole-1-carboximidamide;
3-(2-(Benzyloxy)-4-fluorophenyl)-4-methylene-4,5-dihydro-1H-pyrazole-1-carboximidamide;
(R)-3-(2-(Benzyloxy)-4-fluorophenyl)-4-methyl-4,5-dihydro-1H-pyrazole-1-carboximidamide;
(S)-3-(2-(Benzyloxy)-4-fluorophenyl)-4-methyl-4,5-dihydro-1H-pyrazole-1-carboximidamide;
and salts thereof formed with acids selected from HCl, AcOH, H₂SO₄, H₃PO₄, CF₃CO₂H, and MeS(O)₂OH.

In some embodiments, the inventive method comprises subjecting the compound of Formula 1 or Formula 2 or a pharmaceutically acceptable salt thereof to diastereomeric salt formation with di-O,O'-p-toloyl-D-tartaric acid or di-O,O'-p-toloyl-L-tartaric acid, respectively. Thus, according to a fourth aspect, there is provided a compound selected from the group consisting of:
(R)-3-(4-fluoro-2-hydroxyphenyl)-4-methyl-4,5-dihydro-1H-pyrazole-1-carboximidamide di-O,O'-p-toloyl-D-tartaric acid salt; and
(S)-3-(4-fluoro-2-hydroxyphenyl)-4-methyl-4,5-dihydro-1H-pyrazole-1-carboximidamide di-O,O'-p-toloyl-L-tartaric acid salt.

According to a further aspect, there is provided a pharmaceutical composition comprising a compound according to the second aspect, admixed with one or more pharmaceutically acceptable excipients or carriers. Typically, said excipients are selected from the group comprising filling agents, lubricants, flavours, colourings, sweetenings, buffers, acidifying agents, diluents, and preservatives. Typically, said compositions are administered orally, by oral inhalation, intramuscularly, intravenously, intraperitoneally, or subcutaneously, via implants, rectally, intranasally, or transdermally; preferably orally.

The inventive pharmaceutical composition preferably comprises the compound according to the second aspect in an enantiomerically pure or enantiomerically enriched form.

In some embodiments of the pharmaceutical composition at least 80%, preferably at least 90%, and more preferably at least 95%, of the total content of 3-phenyl-4-methyl-4,5-dihydro-1H-pyrazole-1-carboximidamide derivative in the pharmaceutical composition consists of the R enantiomer.

The compounds of the invention may be used in the prophylaxis and treatment as such, or preferably in a form of a pharmaceutical composition. While it is possible for the active ingredient to be administered alone, it is preferable for it to be present in a pharmaceutical formulation or composition. Accordingly, the invention provides a pharmaceutical composition comprising a compound according to the second aspect, admixed with one or more pharmaceutically acceptable excipients or carriers (collectively referred to herein as "carrier" materials). Pharmaceutical compositions of the invention may take the form of a pharmaceutical formulation as described below.

Exemplary compositions for oral administration include suspensions (including nanosuspensions) which can contain, for example, microcrystalline cellulose for imparting bulk, alginic acid or sodium alginate as a suspending agent, methylcellulose as a viscosity enhancer, and sweeteners or flavoring agents such as those known in the art; and immediate release tablets which can contain, for example, microcrystalline cellulose, dicalcium phosphate, starch, magnesium stearate, calcium sulfate, sorbitol, glucose, and/or lactose and/or other excipients, binders, extenders, disintegrants, diluents, and lubricants such as those known in the art. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, waxes, and the like. Disintegrators include without limitation starch, methylcellulose, agar, bentonite, xanthan gum, and the like. The compounds of Formula 1 or Formula 2 can also be delivered through the oral cavity by sublingual and/or buccal administration. Molded tablets, compressed tablets or freeze-dried tablets are exemplary forms which may be used. Exemplary compositions include those formulating the present compound(s) with fast dissolving diluents such as mannitol, lactose, sucrose, and/or cyclodextrins. Also included in such formulations may be high molecular weight excipients such as celluloses (avicel) or polyethylene glycols (PEG). Such formulations can also include an excipient to aid mucosal adhesion such as hydroxy propyl cellulose (HPC), hydroxy propyl methyl cellulose (HPMC), sodium carboxy methyl cellulose (SCMC), maleic anhydride copolymer (e.g. Gantrez), and agents to control release such as polyacrylic copolymer (e.g. Carbopol 934). Lubricants, glidants, flavors, coloring agents, and stabilizers may also be added for ease of fabrication and use. Lubricants used in these dosage forms include sodium oleate, sodium stearate, magnesium stearate, sodium benzoate, sodium acetate, sodium chloride, and the like. For oral administration in liquid form, the oral drug components can be combined with any oral, non-toxic, pharmaceutically acceptable inert carrier such as ethanol, glycerol, water, and the like.

The pharmaceutical formulations according to the invention include those suitable for oral, parenteral [including subcutaneous, intradermal, intramuscular, intravenous (bolus or infusion), and intraarticular], inhalation (including fine particle dusts or mists which may be generated by means of various types of metered dose pressurized aerosols), nebulizers or insufflators, rectal, intraperitoneal, and topical (including dermal, buccal, sublingual, and intraocular) administration, although the most suitable route may depend upon, for example, the condition and disorder of the recipient.

Formulations of the present invention suitable for oral administration may be presented as discrete units such as capsules, cachets, pills or tablets each containing a predetermined amount of the active ingredient; as a powder or granules; as a solution or a suspension in an aqueous liquid or a non-aqueous liquid, for example as elixirs, tinctures, suspensions (including nanosuspensions) or syrups; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein. The present compounds can, for example, be administered in a form suitable for immediate release or extended release. Immediate release or extended release can be achieved by the use of suitable pharmaceutical compositions comprising the present compounds, or, particularly in the case of extended release, by the use of devices such as subcutaneous implants or osmotic pumps. The present compounds can also be administered liposomally. Preferred unit dosage formulations are those containing an effective dose, as hereinbefore recited, or an appropriate fraction thereof, of the active ingredient.

It should be understood that in addition to the ingredients particularly mentioned above, the formulations of this invention may include other agents conventional in the art having regard to the type of formulation in question, for example those suitable for oral administration may include flavouring agents.

The formulations may conveniently be presented in unit dosage form and may be prepared by any of the methods well known in the art of pharmacy. All methods include the step of bringing the active ingredient into association with the carrier which constitutes one or more accessory ingredients. In general the formulations are prepared by uniformly and intimately bringing into association the active ingredient with liquid carriers or finely divided solid carriers or both and then, if necessary, shaping the product into the desired formulation.

The compounds of the present invention can also be administered in the form of liposome delivery systems, such as small unilamellar vesicles, large unilamellar vesicles, and multilamellar vesicles. Liposomes can be formed from a variety of phospholipids, 1,2-dipalmitoylphosphatidylcholine, phosphatidyl ethanolamine (cephaline), phosphatidylserine, phosphatidylinositol, diphosphatidylglycerol (cardiolipin) or phosphatidylcholine (lecithin).

Formulations for parenteral administration include aqueous and non-aqueous sterile injection solutions which may contain anti-oxidants, buffers, bacteriostats, and solutes which render the formulation isotonic with the blood of the intended recipient; and aqueous and non-aqueous sterile suspensions (including nanosuspensions) which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example saline or water-for-injection, immediately prior to use. Extemporaneous injection solutions and suspensions may be prepared from sterile powders, granules, and tablets of the kind previously described. Exemplary compositions for parenteral administration include injectable solutions or suspensions which can contain, for example, suitable non-toxic, parenterally acceptable diluents or solvents, such as polyethylene glycol, ethanol, 1,3-butanediol, water, Ringer's solution, an isotonic sodium chloride solution, or other suitable dispersing or wetting and suspending agents, including synthetic mono- or diglycerides, and fatty acids, including oleic acid, polysorbates, and Cremaphor.

Exemplary compositions for nasal, aerosol or inhalation administration include solutions in saline, which can contain, for example, benzyl alcohol or other suitable preservatives, absorption promoters to enhance bioavailability, and/or other solubilizing or dispersing agents such as those known in the art.

Formulations for rectal administration may be presented as a suppository with the usual carriers such as cocoa butter, synthetic glyceride esters or polyethylene glycol. Such carriers are typically solid at ordinary temperatures, but liquefy and/or dissolve in the rectal cavity to release the drug.

Formulations for topical administration in the mouth, for example buccally or sublingually, include lozenges comprising the active ingredient in a flavoured basis such as sucrose and acacia or tragacanth, and pastilles comprising the active ingredient in a basis such as gelatin and glycerine or sucrose and acacia. Exemplary compositions for topical administration include a topical carrier such as Plastibase (mineral oil gelled with polyethylene).

The amount of active ingredient which is required to achieve a therapeutic effect will, of course, vary with the particular compound, the route of administration, the subject under treatment, including the type, species, age, weight, sex, and medical condition of the subject and the renal and hepatic function of the subject, and the particular disorder or disease being treated, as well as its severity. An ordinarily skilled physician, veterinarian or clinician can readily determine and prescribe the effective amount of the drug required to prevent, counter or arrest the progress of the condition.

Oral dosages of the present invention, when used for the indicated effects, will range between about 0.01 mg per kg of body weight per day (mg/kg/day) to about 100 mg/kg/day, preferably 0.01 mg per kg of body weight per day (mg/kg/day) to 10 mg/kg/day, and most preferably 0.1 to 5.0 mg/kg/day, for adult humans. For oral administration, the compositions are preferably provided in the form of tablets or other forms of presentation provided in discrete units containing 0.5, 1.0, 2.5, 5.0, 10.0, 15.0, 25.0, 50.0, 100, 500, or 1000 milligrams of the active ingredient for the symptomatic adjustment of the dosage to the patient to be treated. A medicament typically contains from about 0.5 mg to about 1000 mg of the active ingredient. Furthermore, preferred compounds for the present invention can be administered in intranasal form via topical use of suitable intranasal vehicles, or via transdermal routes, using those forms of transdermal skin patches well known to those of ordinary skill in the art. To be administered in the form of a transdermal delivery system, the dosage administration will, of course, be continuous rather than intermittent throughout the dosage regimen.

According to a further aspect, there is provided a compound according to the second aspect, for use as a medicament.

According to a further aspect, there is provided a compound according to the second aspect, for use in treatment of fibrosis, cardiovascular diseases, pain, IBD, inflammatory diseases, or cancer. Typically, said fibrosis is selected from systemic sclerosis, skin fibrosis, liver fibrosis, heart fibrosis, kidney fibrosis, intestinal fibrosis, lung fibrosis including idiopathic pulmonary fibrosis (IPF) and fibrosis associated with pulmonary arterial hypertension (PAH), and fibrosis associated with transplantation, surgery, stenosis, or keloid scarring. Typically, said cardiovascular disease is selected from atherosclerosis and hypertension. Typically, said pain is selected from migraine and pain associated with inflammatory diseases. Typically, said IBD is selected from Crohn's disease and ulcerous colitis. Typically, said cancer is selected from extracellular matrix producing cancers such as breast cancer, pancreas cancer, and liver cancer.

According to a further aspect, there is provided the use of a compound according to the second aspect in the manufacture of a medicament useful in treatment of fibrosis, cardiovascular diseases, pain, IBD, inflammatory diseases, or cancer. Typically, said fibrosis is selected from systemic sclerosis, skin fibrosis, liver fibrosis, heart fibrosis, kidney fibrosis, intestinal fibrosis, lung fibrosis including idiopathic pulmonary fibrosis (IPF) and fibrosis associated with pulmonary arterial hypertension (PAH), and fibrosis associated with transplantation, surgery, stenosis, or keloid scarring. Typically, said cardiovascular disease is selected from atherosclerosis and hypertension. Typically, said pain is selected from migraine and pain associated with inflammatory diseases. Typically, said IBD is selected from Crohn's disease and ulcerous colitis. Typically, said cancer is selected from extracellular matrix producing cancers such as breast cancer, pancreas cancer, and liver cancer.

According to a further aspect, there is provided a method of treating fibrosis, cardiovascular diseases, pain, IBD, inflammatory diseases, or cancer comprising administering a therapeutically effective amount of a compound according to the second aspect to a patient in need thereof. Typically, said fibrosis is selected from systemic sclerosis, skin fibrosis, liver fibrosis, heart fibrosis, kidney fibrosis, intestinal fibrosis, lung fibrosis including idiopathic pulmonary fibrosis (IPF) and fibrosis associated with pulmonary arterial hypertension (PAH), and fibrosis associated with transplantation, surgery, stenosis, or keloid scarring. Typically, said cardiovascular disease is selected from atherosclerosis and hypertension. Typically, said pain is selected from migraine and pain associated with inflammatory diseases. Typically, said IBD is selected from Crohn's disease and ulcerous colitis. Typically, said cancer is selected from extracellular matrix producing cancers such as breast cancer, pancreas cancer, and liver cancer.

The preparation of racemic compounds described in WO2016/207231A1 included 4-nor- and 4-Me-2-pyrazolines, using acetophenones and propiophenones, respectively, as starting materials in the Mannich reaction (Scheme 1).

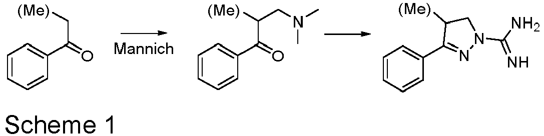

It was found that small amounts of bis-adducts were formed as byproducts in the Mannich reaction with acetophenones, and that the bis-adduct can further react by elimination of dialkylamine to provide a 3-dialkylylamino-2-methylene-propiophenone (Scheme 2).

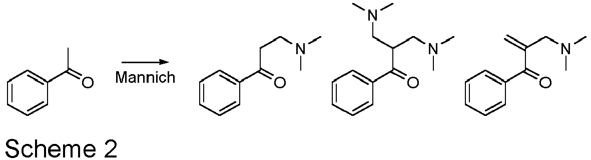

This product can potentially be reacted with aminoguanidine to give a 4-methylene-2-pyrazoline, a suitable substrate for enantioselective hydrogenation, providing chiral products (Scheme 3).

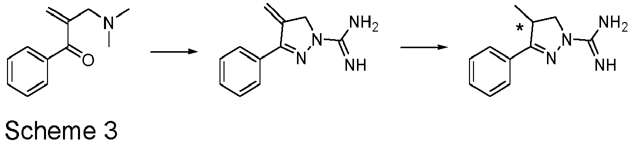

The synthetic plan outlined above was developed into an effective preparative method for the synthesis of (4R)- and (4S)-3-(4-fluoro-2-hydroxyphenyl)-4-methyl-4,5-dihydro-1H-pyrazole-1-carboximidamide, respectively (Scheme 4). It is understood that similar chiral 3-phenyl-4-methyl-4,5-dihydro-1H-pyrazole-1-carboximidamide derivatives can be prepared by basically the same methods.

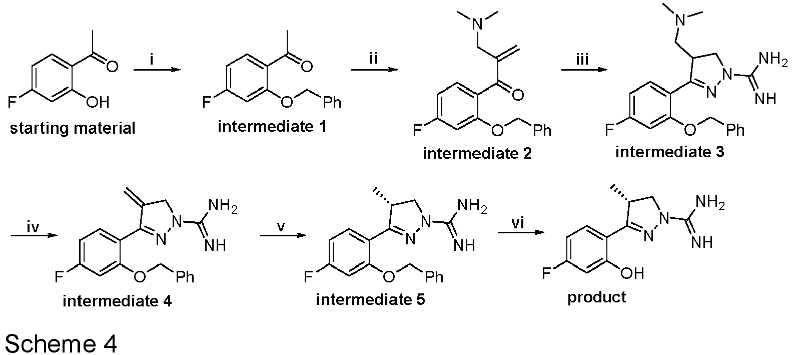

Benzylation (i) - The phenolic moiety of the starting material, 4'-fluoro-2'-hydroxyacetophenone, is protected by benzylation to avoid intramolecular ring-closure with the enone moiety of intermediate 2. Benzylation can be achieved by reacting the phenolic starting material with benzyl bromide in an appropriate solvent like MeCN or DMF in the presence of a base, e.g. K₂CO₃. Alternative protecting groups, stable through the synthetic route ii - v, may be used, e.g. other ethers and silyl ethers.

Mannich reaction (ii) - This reaction, a "double Mannich reaction", provides two - CH₂NMe₂ moieties, one of which can eliminate HNMe₂, to give the enone structure. The starting acetophenone can be reacted with ready-made Mannich reagents, e.g. the Eschenmoser's salt (H₂C=NMe₂⁺ I⁻), or alternatively, with *in situ* generated reagents prepared under acidic conditions from a formaldehyde equivalent, e.g. paraformaldehyde or 1,3-dioxolane, and a dialkylamine or a dialkylammonium salt, e.g. pyrrolidine or H₂NMe₂Cl, respectively.

Ring-closure with aminoguanidine (iii) - Most conveniently, aminoguanidine hydrochloride is used in the ring-closure, which can be performed both under acidic, e.g. in refluxing EtOH, and basic conditions, and in aqueous or non-aqueous solvents, typically at temperatures ranging from room temperature to 80 °C. Under basic conditions, aq. NaOH can be used as base in solvents like THF, MeCN, EtOH and water. The intermediate 3 is most conveniently isolated as a salt, e.g. as a bis-trifluoroacetic acid (bis-TFA) salt or bis-methansulfonic acid salt, which can be used as starting material in the following Cope-elimination.

Cope-elimination (iv) - This reaction is an oxidation-elimination process, wherein the trialkylamine moiety is oxidized to give an N-oxide, which eliminates N-hydroxy-dialkylamine to provide the exo-methylene moiety. Per-acids, e.g. MCPBA, or inorganic oxides, e.g. potassium peroxymonosulfate, are often used as oxidation agents, optionally generated *in situ* by oxidation with e.g. H₂O₂ or O₂ (g). (Xingwei Cai et al., Asian Journal of Chemistry; Vol. 24, No. 9 (2012), 3781-3784). The N-oxide of intermediate 3 can be isolated before elimination, or the two-step process can be performed as a one-pot procedure. The elimination process is affected by heating the N-oxide in an appropriate solvent, e.g. EtOAc, MeOH or CH₂Cl₂. Temperatures vary with the substrates and solvents. For the N-oxide of intermediate 3, elimination occurs conveniently at 40-80 °C.

Another option for the elimination processes is via N-alkylation to give a tetraalkylammonium salt that can eliminate trialkyl-amine under basic conditions.

Enantioselective hydrogenation (v) - Transition metal catalysis for asymmetric hydrogenation of double bonds, e.g. a C=C double bond, using hydrogen gas may be a powerful way to generate chiral products (Biosca, M. et al. Asymmetric hydrogenation in industry, Advances in Catalysis, Volume 68, 2021, Pages 341-383). Rh and Ru in complex with chiral diphosphine ligands are among the most common catalysts and many ligands are commercially available in both enantiomeric forms, providing access to both R- and S-enantiomeric products. Normally, many different reaction conditions, including the catalyst system, are tested to identify adequate and scalable preparative methods that can provide an acceptable chiral purity of the product. In addition to the selection of metal source and ligand, catalyst loading, concentration, solvent(s), additives, temperature, and hydrogen pressure should also be considered. For basic compounds, salt formation by addition of acids may have a large impact on the results. Alternatively, the preformed salt of the staring material can be used in the reaction.

Asymmetric hydrogenation of intermediate 4 can be performed using a catalyst prepared from a Rh source, e.g. [Rh(nbd)₂]/BF₄ (bis(norbornadiene)rhodium(I) tetrafluoroborate), and a diphosphine ligand, e.g. SL-A242, SL-WO09, SL-J004, SL-J006, SL-J007, SL-J502, SL-J505, SL-J212, SL-C008, SL-M002, or SL-T002, ligands which all are commercially available in both enantiomeric forms. The intermediate 4 is preferably used as a salt form, formed from e.g. the acids AcOH, CF₃CO₂H (TFA), MeSO₂OH, H₂SO₄, KHSO₄, or H₃PO₄ or alternatively in combination with an acid as an additive, in an appropriate solvent, e.g. MeOH, EtOH, I-PrOH, THF, 2-Me-THF or dioxane.

Debenzylation (vi) - Catalytic hydrogenation in inert solvents, e.g. alcohols, THF or toluene, with Pd/C as catalyst and H₂ (g) as hydrogen source is a standard procedure. Alternative hydrogen sources are e.g. ammonium formate and c-hexadiene.

Debenzylation of intermediate 5 can also be performed under acidic solvolytic conditions, e.g. in aq. conc. HCl mixtures with MeOH, EtOH or AcOH or in HBr/AcOH.

The chiral purity of the target product can, if desired, be further improved by recrystallization of intermediate 5 and/or the product, preferably as salt derivatives, e.g. HCl salts, or as diastereomeric salts, e.g the di-O,O'-p-toloyl-D-tartaric acid (D-DpTTA) salt of the product. Both the D- and the L-isomer of the di-O,O'-p-toloyl-tartaric acid, (2S, 3S) and (2R, 3R), respectively, are commercially available. The L-isomer can thus be used to improve the chiral purity of the (4S)-isomer of the product.

The present invention will now be described in more detail by the following examples, which are included in order to disclose certain embodiments of the invention, but not in any way to limit the scope of the invention.

### Examples

### Example 1

### 1-(2-(Benzyloxy)-4-fluorophenyl)ethan-1-one

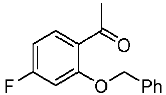

A mixture of 4'-fluoro-2'-hydroxyacetophenone (46.2 g, 0.30 mol), benzyl bromide (51.3 g, 0.30 mol), and K₂CO₃ (81.2 g, 0.60 mol) in MeCN (250 mL) was stirred at reflux temperature (b.p. 82 °C) for 2 h. After cooling, the reaction mixture was filtered and the filtrate was concentrated at reduced pressure to give the title compound as a white solid (72.0 g, 98%).

¹H-NMR (CD₃OD) d 2.52 (s, 3H), 5.22 (s, 2H), 6.77 (dt,1H), 7.01 (dd, 1H), 7.35 (t, 1H), 7.41 (t, 2H), 7.50 (d, 2H), 7.78 (dd, 1H).

### Example 2

### 1-(2-(Benzyloxy)-4-fluorophenyl)-2-((dimethylam ino)methyl)prop-2-en-1-one

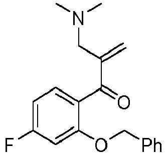

1,3-Dioxolane as formaldehyde equivalent:
A mixture of 1-(2-(benzyloxy)-4-fluorophenyl)ethan-1-one (14.7 g, 60.2 mmol), dimethylamine hydrochloride (49.1 g, 602 mmol) and conc HCl (37%, 0.6 mL) in 1,3-dioxolane (250 mL) was stirred at 86 °C overnight. After cooling, the reaction mixture was partitioned between water (250 mL) and MTBE (200 mL). The aqueous phase was basified by adding a solution of K₂CO₃ (75 g) in water (50 mL) and was extracted with methyl-tert-butyl ether (2 x 350 mL). The organic phase was washed with water, dried (Na₂SO₄), filtered, and concentrated at reduced pressure to give the title compound as an oil (18.0 g, 95 %).

¹H-NMR (CDCl₃) d 2.18 (s, 6H), 3.23 (s, 2H), 5.05 (s, 2H), 5.80 (s, 1H), 6.00 (d, 1H), 6.67-6.74 (m, 2H), 7.29-7.45 (m, 6H).

Paraformaldehyde as formaldehyde equivalent:

A mixture of 1-(2-(benzyloxy)-4-fluorophenyl)ethan-1-one (4.0 g, 16.4 mmol), dimethylamine hydrochloride (5.3 g, 64 mmol), paraformaldehyde (2.14 g, 64 mmol) and AcOH (8.0 mL) in iso-PrOAc (20 mL) was stirred under N₂ (g) at 90 °C overnight. After cooling, the reaction mixture was concentrated at reduced pressure, diluted with methyl-tert-butyl ether (40 mL) and basified (pH about 10) by addition of aq 2M K₂CO₃ (60 mL). The organic phase was separated and washed with water, dried (Na₂SO₄), filtered, and concentrated at reduced pressure to give the title compound as an oil (4.75 g, 93 %).

### Example 3

### 3-(2-(Benzyloxy)-4-fluorophenyl)-4-((dimethylamino)methyl)-4,5-dihydro-1H-pyrazole-1-carboximidamide bis-trifluoroacetic acid salt

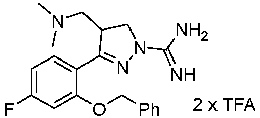

An aq. solution of NaOH (5M, 11.1 mL) was added to a solution of aminoguanidine hydrochloride (6.19 g, 55 mmol) in water (33 mL) and EtOH (100 mL) at 0°C. The combined solutions were added to 1-(2-(benzyloxy)-4-fluorophenyl)-2-((dimethylamino)methyl)prop-2-en-1-one (13.3 g, 42,4 mmol) and the mixture was stirred at room temperature overnight and then portioned between EtOAc and brine. The organic phase was separated, dried (Na₂SO₄) and concentrated at reduced pressure. The residue (ca 19 g) was dissolved in EtOAc (100 mL) and trifluoroacetic acid (6.34 mL, 83 mmol) was added. Petroleum ether (100 mL) was slowly added to precipitate the product. The mixture was stirred for 5 h and the product was collected by filtration, washed with EtOAc and dried to give the title compound (11.9 g, 50%) as a white solid.

¹H-NMR (CD₃OD) d 2.59 (broad s, 6H), 3.19 (dd, 1H), 3.28-3.35 (m, 1H, solvent at 3,31 ppm), 4.11 (dd, 1H), 4.25 (t, 1H), 4.53 (m, 1H), 5.24 (dd, 1H), 5.29 (dd, 1H), 6.86 (dt,1H), 7.14 (dd, 1H), 7.38-7.47 (m, 3H), 7.54 (d 2H), 7.91 (dd, 1H).

### Example 4

### 3-(2-(Benzyloxy)-4-fluorophenyl)-4-methylene-4,5-dihydro-1H-pyrazole-1-carboximidamide trifluoroacetic acid salt

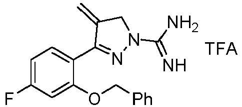

Oxone (3.33 g, 5,42 mmol) was added to a mixture of 3-(2-(benzyloxy)-4-fluorophenyl)-4-((dimethylamino)methyl)-4,5-dihydro-1H-pyrazole-1-carboximidamide bis-trifluoroacetic acid salt (4.99 g, 8.86 mmol) and Na₂HPO₄(H₂O)₂ (36.9 mmol, 6.57 g) in EtOAc (80 mL) and water (20 mL). After the addition, the slurry dissolved, and the mixture was stirred vigorously at room temperature for 20 min giving a two-phase system. Na₂SO₄ (26.7 g) was added and stirring was continued for 10 min. The clear organic phase (containing the N-oxide) was separated, and the aqueous/solid residue was extracted with EtOAc (2x20 mL). The combined organic phases were concentrated to a volume of about 80 mL for azeotropic removal of water. The solution was stirred at 60°C for 50 min. After cooling, trifluoroacetic acid (0.4 mL) was added to the stirred solution, followed by addition of petroleum ether (40 mL) and water (40 mL) to give a white precipitate. After stirring vigorously for 5 min, the solids were collected by filtration, washed with water and EtOAc/petroleum ether (1:1), and dried to give the title compound as white crystals (3.59 g, 96%).

¹H-NMR (CD₃OD) d 4.75 (t, 2H), 5.13 (s, 2H), 5.33 (t, 1H), 5.57 (t, 1H), 6.84 (dt, 1H), 7.06 (dd, 1H), 7.29-7.44 (m, 6H).

### Example 5

### 3-(2-(Benzyloxy)-4-fluorophenyl)-4-methylene-4,5-dihydro-1H-pyrazole-1-carboximidamide

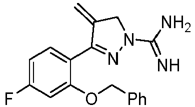

1 M aq. NaOH (9.0 mL, 9.0 mmol) was added to a solution of 3-(2-(benzyloxy)-4-fluorophenyl)-4-methylene-4,5-dihydro-1H-pyrazole-1-carboximidamide trifluoroacetic acid salt (3.59 g, 8.5 mmol) in MeOH (36 mL). The solution was then cooled and stirred on an ice bath. Water (18 mL) was added to precipitate the product, which was collected by filtration after stirring for 10 min. The product was washed with MeOH/water (1:3) and water and dried to give the title compound (2.24 g, 81%).

¹H-NMR (CD₃OD) d 4.65 (t, 2H), 5.08 (t, 1H), 5.11 (s, 2H), 5.34 (t, 1H), 6.79 (dt, 1H), 6.99 (dd, 1H), 7.27-7.41 (m, 6H).

### Example 6

### (R)-3-(2-(Benzyloxy)-4-fluorophenyl)-4-methyl-4,5-dihydro-1H-pyrazole-1-carboximidamide

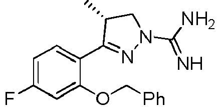

0,04% eq [Rh(nbd)₂]/SL-J212-1 (CAS RN^{®} 849924-41-0) (ratio: 1/1.2) as catalyst. A mixture of 3-(2-(benzyloxy)-4-fluorophenyl)-4-methylene-4,5-dihydro-1H-pyrazole-1-carboximidamide (1 eq, 0.12 M), methane sulfonic acid (2.6 eq) and the catalyst in THF/MeOH (9:1) was stirred under hydrogen gas (15 bar) overnight. Chiral HPLC analysis showed full conversion and enantiomeric R/S ratio of 93:7. The reaction mixture is concentrated at reduced pressure and the product isolated by conventional basic two-phase work-up to give the title compound.

### Example 7

### (R)-3-(2-(Benzyloxy)-4-fluorophenyl)-4-methyl-4,5-dihydro-1H-pyrazole-1-carboximidamide hydrochloride

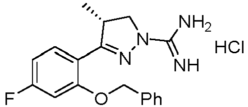

A solution of HCl in EtOH (1-2 eq) is added to a solution of (R)-3-(2-(benzyloxy)-4-fluorophenyl)-4-methyl-4,5-dihydro-1H-pyrazole-1-carboximidamide in EtOH/EtOAc. The product is precipitated by addition of i-PrOAc and collected by filtration.

### Example 8

### (R)-3-(4-fluoro-2-hydroxyphenyl)-4-methyl-4,5-dihydro-1H-pyrazole-1-carboximidamide hydrochloride

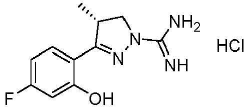

(R)-3-(2-(Benzyloxy)-4-fluorophenyl)-4-methyl-4,5-dihydro-1H-pyrazole-1-carboximidamide hydrochloride is debenzylated by Pd/C catalysed hydrogenation by stirring in MeOH under H₂ (g) overnight. After filtration, the filtrate is concentrated, and the product is precipitated by addition of EtOAc/i-PrOAc and collected by filtration.

### Example 9

### (R)-3-(4-fluoro-2-hydroxyphenyl)-4-methyl-4,5-dihydro-1H-pyrazole-1-carboximidamide D-DpTTA salt

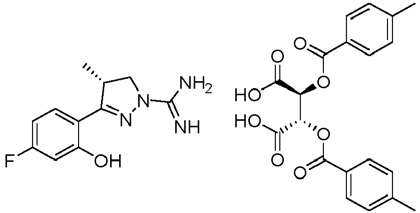

Di-O,O'-para-toloyl-D-tartaric acid (1 eq) is added to a solution of (R)-3-(2-(benzyloxy)-4-fluorophenyl)-4-methyl-4,5-dihydro-1H-pyrazole-1-carboximidamide (R:S about 92:8) in EtOH/EtOAc. The product is precipitated by addition of i-PrOAc and collected by filtration. The salt (0.5 g) was triturated in EtOH (10 mL) and i-PrOAc (5 mL) at 50°C overnight. After cooling, the salt was collected by filtration to give a product with increased chiral purity (62%, R:S 98.7: 1.3).

### Example 10

### (R)-3-(4-fluoro-2-hydroxyphenyl)-4-methyl-4,5-dihydro-1H-pyrazole-1-carboximidamide hydrochloride

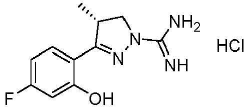

Aq. conc. HCl (3.06 kg) was added to a mixture of (R)-3-(4-fluoro-2-hydroxyphenyl)-4-methyl-4,5-dihydro-1H-pyrazole-1-carboximidamide di-O,O'-para-toloyl-D-tartaric acid salt (17.5 kg, mol) in EtOH (265 L) and the solution was stirred for 1 h to form a clear solution. The solution was concentrated to approx. 90 L The HCl salt was precipitated by addition of i-PrOAc (265 L). After stirring for 1 h at room temperature, and then at 5 °C overnight, the product was collected by filtration, washed with i-PrOAc and dried to give the title compound (6.70 kg, 87%). ¹H-NMR ((CD₃)₂SO) d 1.16 (d, 3H), 3.69 (dd, 1H), 4.04-4.17 (m, 2H), 6.77 (dt, 1H), 6.88 (dd, 1H), 7.76 (dd, 1H), 7.89 (broad s, 4H), 10.61 (broad s, 1H).

## Claims

1. A method for preparing a compound of Formula 1 or Formula 2 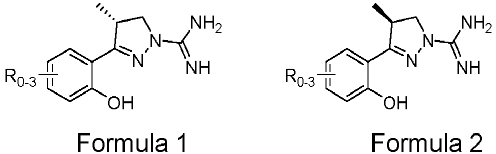
or a pharmaceutically acceptable salt thereof;
wherein R₀₋₃ represents 0-3 aromatic substituents independently selected from methyl, methoxy, F, and Cl;
the method comprising:
a) providing a compound of Formula 3 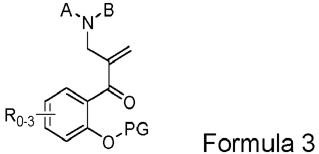 wherein
A and B are selected from methyl, ethyl, benzyl, or together form a pyrrolidine or piperidine N-heterocycle;
R₀₋₃ represents 0-3 aromatic substituents independently selected from methyl, methoxy, F, and Cl;
PG is selected from methyl, benzyl, 4-methoxybenzyl, and tert-butyl-dimethylsilyl;
b) subjecting the compound of Formula 3 to a ring closure process with aminoguanidine or aminoguanidine hydrochloride to obtain a compound of Formula 4, optionally as a salt with an acid selected from HCl, AcOH, H₂SO₄, H₃PO₄, CF₃CO₂H and MeS(O)₂OH, 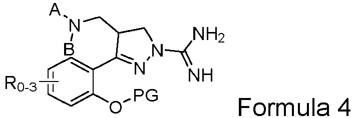 wherein A, B, R₀₋₃, and PG are as described above;
c) subjecting the compound of Formula 4 to an elimination process to obtain a compound of Formula 5, optionally as a salt with an acid selected from HCl, AcOH, H₂SO₄, H₃PO₄, CF₃CO₂H and MeS(O)₂OH 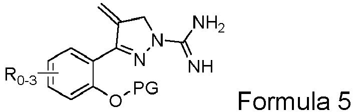 wherein R₀₋₃, and PG are as described above;
d) subjecting the compound of Formula 5 to enantioselective hydrogenation to obtain a compound of Formula 6 or Formula 7, optionally as a salt with an acid selected from HCl, AcOH, H₂SO₄, H₃PO₄, CF₃CO₂H and MeS(O)₂OH; and 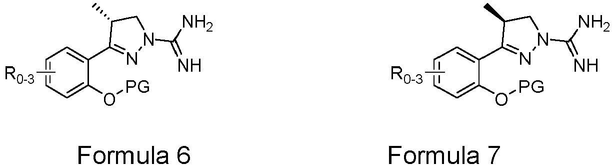 wherein R₀₋₃, and PG are as described above;
e) subjecting the compound of Formula 6 or Formula 7 to deprotection, and optionally forming a pharmaceutically acceptable salt of the deprotected compound to obtain the compound of Formula 1 or Formula 2 or a pharmaceutically acceptable salt thereof.

2. The method according to claim 1, further comprising:
f) subjecting the compound of Formula 1 or Formula 2 or a pharmaceutically acceptable salt thereof obtained in e) to diastereomeric salt formation with di-O,O'-p-toloyl-D-tartaric acid or di-O,O'-p-toloyl-L-tartaric acid, respectively, followed by salt exchange to obtain the compound of Formula 1 or Formula 2 or a pharmaceutically acceptable salt thereof.

3. The method according to any one of the preceding claims, wherein the obtained compound of Formula 1 or Formula 2 or a pharmaceutically acceptable salt thereof has an enantiomeric purity of at least 80%, preferably at least 90%, and more preferably at least 95%.

4. A compound of Formula 1 or Formula 2 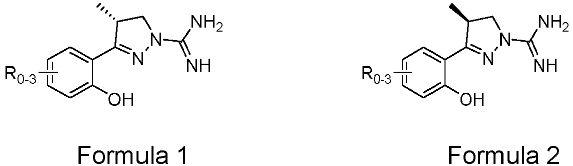
or a pharmaceutically acceptable salt thereof;
wherein R₀₋₃ represents 0-3 aromatic substituents are independently selected from methyl, methoxy, F, and Cl.

5. A compound according to claim 4, wherein the compound of Formula 1 or Formula 2 or a pharmaceutically acceptable salt thereof has an enantiomeric purity of at least 80%, preferably at least 90%, and more preferably at least 95%.

6. A compound according to any one of claims 4-5, wherein
R₀₋₃ represents 0-2 aromatic substituents independently selected from F, and Cl.

7. A compound according to any one of claims 4-6, wherein the compound is selected from: 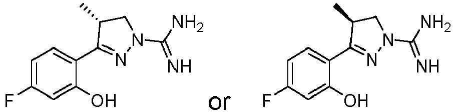 or a pharmaceutically acceptable salt thereof.

8. A compound according to any one of claims 4-7, wherein the compound is:
(R)-3-(4-fluoro-2-hydroxyphenyl)-4-methyl-4,5-dihydro-1H-pyrazole-1-carboximidamide hydrochloride; or
(S)-3-(4-fluoro-2-hydroxyphenyl)-4-methyl-4,5-dihydro-1H-pyrazole-1-carboximidamide hydrochloride.

9. A compound selected from Formula 3, Formula 4, Formula 5, Formula 6, or Formula 7 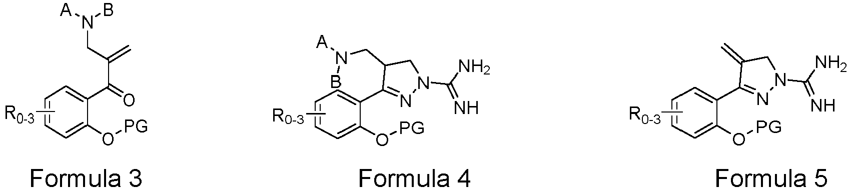 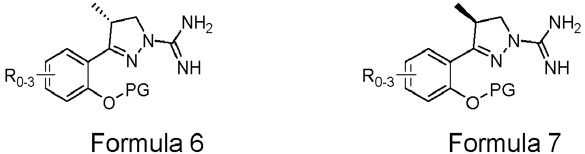
and salts thereof formed with acids selected from HCl, AcOH, H₂SO₄, H₃PO₄, CF₃CO₂H, and MeS(O)₂OH; wherein
A and B are selected from methyl, ethyl, benzyl, or together form a pyrrolidine or piperidine N-heterocycle;
R₀₋₃ represents 0-3 aromatic substituents independently selected from methyl, methoxy, F, and Cl; and
PG is selected from methyl, benzyl, 4-methoxybenzyl, and tert-butyl-dimethylsilyl.

10. A compound according to claim 9, wherein the compound is selected from Formula 3 or Formula 4, and salts thereof formed with acids selected from HCl, AcOH, H₂SO₄, H₃PO₄, CF₃CO₂H, and MeS(O)₂OH; wherein
A and B are selected from methyl, and benzyl;
R₀₋₃ represents 0-3 aromatic substituents independently selected from F, and Cl; and
PG is benzyl.

11. A compound according to claim 9, wherein the compound is selected from: 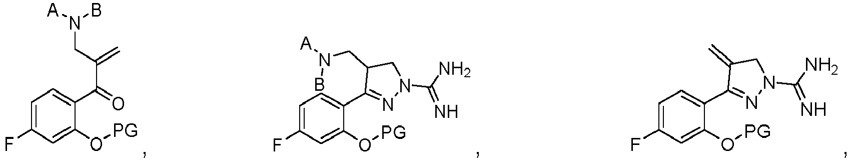 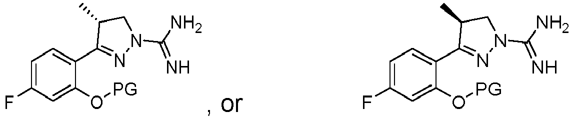
and salts thereof formed with acids selected from HCl, AcOH, H₂SO₄, H₃PO₄, CF₃CO₂H, and MeS(O)₂OH; wherein
A and B are selected from methyl, ethyl, benzyl, or together form a pyrrolidine or piperidine N-heterocycle; and
PG is selected from methyl, benzyl, 4-methoxybenzyl, 4-methoxybenzyl, and tert-butyl-dimethylsilyl.

12. A compound according to claim 9, wherein the compound is selected from:
1-(2-(Benzyloxy)-4-fluorophenyl)-2-((dimethylamino)methyl)prop-2-en-1-one;
3-(2-(Benzyloxy)-4-fluorophenyl)-4-((dimethylamino)methyl)-4,5-dihydro-1H-pyrazole-1-carboximidamide;
3-(2-(Benzyloxy)-4-fluorophenyl)-4-methylene-4,5-dihydro-1H-pyrazole-1-carboximidamide;
(R)-3-(2-(Benzyloxy)-4-fluorophenyl)-4-methyl-4,5-dihydro-1H-pyrazole-1-carboximidamide;
(S)-3-(2-(Benzyloxy)-4-fluorophenyl)-4-methyl-4,5-dihydro-1H-pyrazole-1-carboximidamide;
and salts thereof formed with acids selected from HCl, AcOH, H₂SO₄, H₃PO₄, CF₃CO₂H, and MeS(O)₂OH.

13. A compound selected from the group consisting of:
(R)-3-(4-fluoro-2-hydroxyphenyl)-4-methyl-4,5-dihydro-1H-pyrazole-1-carboximidamide di-O,O'-p-toloyl-D-tartaric acid salt; and
(S)-3-(4-fluoro-2-hydroxyphenyl)-4-methyl-4,5-dihydro-1H-pyrazole-1-carboximidamide di-O,O'-p-toloyl-L-tartaric acid salt.

14. A pharmaceutical composition comprising a compound according to any one of claims 4-8, admixed with one or more pharmaceutically acceptable excipients or carriers.

15. A compound according to any one of claims 4-8 for use as a medicament.

16. A compound according to any one of claims 4-8, for use in treatment of fibrosis, cardiovascular diseases, pain, IBD, inflammatory diseases, or cancer.

17. Use of a compound according to any one of claims 4-8 in the manufacture of a medicament useful in treatment of fibrosis, cardiovascular diseases, pain, IBD, inflammatory diseases, or cancer.

18. A method of treating fibrosis, cardiovascular diseases, pain, IBD, inflammatory diseases, or cancer comprising administering a therapeutically effective amount of a compound according to any one of claims 4-8 to a patient in need thereof.
